# EUROPEAN PATENT SPECIFICATION

(11) **EP 0 616 541 B1**
(45) Date of publication and mention of the grant of the patent: **25.11.1998**
(21) Application number: 93923194.0
(22) Date of filing: 30.09.1993
(51) Int. Cl.: A61M 5/20, A61M 37/00, A61M 5/178, A61M 5/00

(54) **MEDICAMENT INJECTORS AND METHODS**
MEDIZINISCHE SPRITZE UND METHODE
INJECTEURS DE MEDICAMENTS ET PROCEDES

(30) Priority: 05.10.1992 US 956952
(43) Date of publication of application: 28.09.1994
(73) Proprietor: SENETEK PLC, Maryland Heights, MO 63043 (US)
(72) Inventor: WACKS, Jonathan, L., Forest Hills, NY 11375 (US)
(74) Representative: Bayliss, Geoffrey Cyril
(86) International application number: US9309376
(87) International publication number: WO9407553

(56) References cited:
- WO-A-93/02720
- US-A- 3 066 670
- US-A- 3 136 313
- US-A- 3 182 660
- US-A- 3 368 558
- US-A- 3 702 609
- US-A- 3 742 948
- US-A- 4 394 863
- US-A- 4 767 413
- US-A- 5 102 393

## Description

The present invention relates to injection/aspiration devices, injection devices, and to cartridge vials for use with either or both, all for medical and research purposes and more particularly to devices designed for injecting medicines and other fluids into human beings and other subjects using a hollow injection needle.

The injection/aspiration component and some of the cartridge vial components of the invention are also configured to aspirate blood and/or other fluids. A mechanical actuator device of the invention can be adapted to use with many of the cartridges of the invention.

In the conventional medical procedure for injecting medicines or for aspirating blood or fluids, a syringe with a hollow injection/aspiration needle, such as a standard hypodermic needle, is used. Needles are repugnant to many patients, particularly those who must have regular injections of medicine or blood samples taken. Among these are elderly patients and people who must have daily injections of insulin or other drugs. Similarly, pediatric patients are particularly afraid of needles. Exposed or visible needles are undesirable because they create fear and apprehension in many subjects.

Another problem in the medical field is that of the communication of infectious diseases caused by used needles and syringes and fluids therefrom coming into contact with doctors, nurses, or other medical personnel.

Needle tips often remain exposed after aspiration of a fluid or blood from a subject, or after injection of a medicine into a subject and medical personnel are sometimes accidentally pricked with such tips. This problem is particularly acute in situations where a syringe and needle have been contaminated with particularly virulent organisms such as the AIDS virus or the hepatitis virus. The risk of puncture with a contaminated needle point is of particular concern after an injection because a finger, hand or other part of the person administering the injection is typically in close physical proximity to the needle during its removal from the subject's tissues, during replacement of a needle or the needle cover or during removal of the needle from a syringe for disposal.

There is also danger of such exposures to personnel, such as maintenance people, other than medical personnel, when a used needle and/or syringe is laid aside or discarded with a needle tip still exposed. This danger continues even when a used needle and/or syringe are placed in a disposal container. For instance, it is a routine medical procedure to use a device which cuts off the tip of an exposed needle so that it may not be reused. However, this procedure still leaves exposed needle stubs and syringe parts which may be contaminated with infectious agents and with which persons may come into contact and be infected. Thus, it is not uncommon for discarded needle stubs to protrude through plastic garbage bags or other containers and present serious risk of a puncture wound to a person handling or otherwise coming into contact with the container. Similarly, even after used needles are removed from syringes and placed in sealed containers, the exposed syringes must also be placed in sealed containers to reduce the likelihood of infectious contact with personnel. Sanitary disposal of used needles and used syringes is an expensive and time consuming process and entails significant risk of exposure to infectious disease vectors.

As is known in the art, many injection needles are provided with removable covers which can be removed prior to an injection and replaced onto the needle after an injection to prevent unwanted needle punctures. The act of replacment of a needle cover exposes the user to additional risk of puncture because the aperture end of conventional needle covers offers little surface area around the aperture which would shield the user from the needle.

A related problem is that of the dangers of exposing a needle to the atmosphere prior to its being used in giving an injection or withdrawing a body fluid. Not only is there danger of wounds to user personnel and patients from the exposed needle tip, but also there is the danger that the exposed needle will become contaminated by airborne or aerosol born microbial and other contaminants and infect the patient eventually injected. This danger is particularly acute in hospitals and other medical treatment areas where strains of antibiotic-resistant microbes endemically contaminate the air and all exposed surfaces. Contact with non-sterile air is a certainty with conventional, exposed-needle, syringe technology because, in this technology, needles are routinely exposed to the air or surfaces for some discrete amount of time during use. Also, in emergency use situations such as military combat, natural disasters, or industrial accidents, the unused needle may be left exposed to such contaminants by untrained, harried or inexperienced personnel.

A related problem arises in certain prior art devices such as US 5,102,393 in which the needle is fixed to an ampule containing fluid medicament to be injected. Although the needle is enclosed within the confines of an injection device, as long as it is fixed to the ampule it represents a source of potential contamination of the fluid medicament by microbes that may accompany the needle.

Yet another common problem in conventional syringe/injection technology is that relating to improperly given injections. The differences in the rate which a needle must travel during insertion into and withdrawal from the subject's tissues and the rate at which a syringe piston must be operated in order to inject or aspirate fluid in a painless manner are substantial. The techniques of various medical personnel in using conventional syringes are varied. Techniques vary according to the position of the subject, how that portion of the subject's anatomy which is to be injected is held, and by the various individual techniques of medical personnel. This problem is particularly acute with respect to untrained or inexperienced personnel. When a needle is inserted too slowly, needless pain results. These problems are overwhelmingly due to the difficulty of operating a syringe and needle in a manner which appropriately varies the rates of needle insertion and withdrawal and the rates of fluid injection and aspiration.

An additional problem in the field is that of dosage management. For subjects who give themselves injections, either because they require regular doses of injected pharmaceuticals or because medical personnel are not available, it is critical to insure that dosages are correct. Diabetic subjects, particularly diabetics who suffer from the related condition of blindness, often find themselves in such situations. Other blind people are similarly in need of a product which insures that both the type of medication and its dosage are correct for their specific needs. Similarly, soldiers in the field, travelers requiring regular injectable medications, and subjects in emergency situations where self-injection is necessary often have difficulty administering the proper dosage of a given drug and often have difficulty in using a conventional syringe. Such problems are also compounded by darkness or poor lighting conditions in battlefield, power failure, and other crisis or emergency situations.

It is well known in the medical injection field that, when administering a drug or other substance intramuscularly, an attempt is made to aspirate blood or other physiological fluid after insertion of the injection needle into a site thought to be suitable. Such an attempted aspiration is made in order to interrogate the injection site (i.e., essentially, as understood in the art the volume of tissue immediately adjacent to and in fluid communication with the open end of the needle) for the presence of blood, lymph, cerebrospinal fluid, or the like. This interrogation is made to insure that pharmaceutical substances or other fluids are not unintentionally injected inappropriately, e.g., into a blood vessel, lymph vessel or into cerebrospinal fluid. The inappropriate administration of a drug or other substance into a blood vessel, lymph vessel or cerebrospinal cavity could result in any of a number of adverse effects including nausea, unwanted toxicity, paralysis, neurological damage or even death. Moreover, the administration of pharmaceutical substances to inappropriate sites often results in attenuation or loss of the substance's desired, specific functional characteristics or activities. Thus, it is of paramount importance to insure that a needle used in administering a drug or other substance be inserted into an appropriate injection site.

For subjects injecting themselves at home, in emergency situations, or in combat, it is virtually impossible to perform the correct procedure. Similarly, personnel who are untrained in medical injection procedures but must give injections because of crisis or emergency situations are much more likely to incorrectly administer a substance, either by administering an incorrect dosage or administering by placing the needle so that its tip is positioned at an inappropriate site (e.g., so that the substance is injected directly into the blood stream rather than intramuscularly).

An example where a pharmaceutical substance must be delivered to an exact site is in the administration of certain chemotherapeutic drugs for the treatment of cancer. It is essential that chemotherapeutic drugs be delivered to the exact target tissue. Exposure of some of these drugs to the skin or to the incorrect tissue or to the bloodstream may cause severe side effects. It is thus desirable to have a means for automatically interrogating the fluids in an injection device to insure that blood or other physiological fluids are not being inappropriately aspirated from a possible injection site into the device before drug is administered with the device.

Thus, it would also be desirable to have a device which will automatically prevent injection of pharmaceuticals at an undesirable or inappropriate injection site. In other cases, however, it might be desirable or necessary to inject a drug into, for example, the bloodstream; then the interrogation would be to assure that a physiological fluid, such as blood, is aspirated into the injection device before using it to inject the drug.

In those circumstances where aspiration and interrogation are unnecessary, it is still desirable to maintain sterility of the injection needle and medicament prior to the injection, and to automatically inject a proper dose of fluid. Mechanically powered automatic injectors are desirable in situations where motorized injectors are too susceptible to battery or electrical system failure.

Mechanically powered automatic injectors are also desirable where longterm storage would prohibit the use of or decrease the effectiveness of motorized or solenoid powered injectors or other injectors requiring electrical power. Moreover, the minimum cost of manufacture of mechanical injectors of straightforward design is an important advantage over more complex designs and over designs requiring electrical components and circuitry.

### Summary and Objects of the Invention

It is an object of the present invention to provide concealment of an injection needle at all stages of use, thereby reducing the apprehension of the patient.

It is similarly an object of the present invention to maintain sterility of an injection needle at all stages of use by providing means whereby the needle, prior to contact with the skin of a patient for penetration therethrough, is never exposed to any potentially contaminating surfaces, aerosols or airborne particles or microbes.

It is another object of the present invention to provide an injection vial which eliminates the dangers of infection or injury resulting from accidental contact with exposed needles.

It is still another object of the present invention to provide a sanitarily disposable injection vial with a needle which, after use in an injection, retracts completely into said vial to reduce the risk of disease transmission caused by the risk of exposure to a contaminated needle or to a contaminated syringe or to parts thereof.

It is yet another object of the present invention to provide a sanitarily disposable injection vial which can be safely discarded without the need for special equipment or containers and which can be safely and sanitarily disposed of in non-hospital, rugged, or emergency environments.

It is also an object of the present invention to provide an ampule or cartridge for dispensing fluid medication which can be filled by the use of conventional pharmaceutical packaging machinery, thus avoiding the substantial expense which would be required for the development and production of non-conventional packaging methods and machinery.

It is a further object of the present invention to reduce the risk of an improperly administered injection by providing means for precisely, automatically, and programmably controlling the rate of needle insertion, the rate of needle withdrawal, the rate of medicine injection, and the rate of fluid aspiration.

It is also an object of the present invention to reduce the risk that an incorrect dosage of a fluid medicine will be administered by injection.

It is a further object of the invention to reduce the risk that an incorrect drug will be administered to a subject or be self-administered by a subject.

It is an object of the present invention to provide means for the automatic interrogation of an injection site to determine whether or not the injection needle tip is at an appropriate or desired site and in the desired target tissue.

It is a further object of the present invention to provide means for automatically scanning an injection/aspiration cartridge during operation to determine if blood or other physiological fluids have been aspirated from an actual or possible injection site into the cartridge.

It is also an object of the present invention to provide means for automatically preventing the injection of medicines or other fluids into an undesired or inappropriate tissue or site.

Furthermore, it is an object of the present invention to provide means to administer a drug to a pre-selected target tissue and avoid exposure of the drug to tissues which would be undesirably damaged by such exposure.

It is yet another object of the present invention to provide a means for giving or self-administering penile injections of drugs with a minimum of pain and apprehension on the part of the male.

It is an additional object of the invention to provide a mechanically powered injector which needs no electrical power or circuitry and which can be stored for long periods of time.

It is a similar object of the invention to provide a mechanically powered injector which is not suitable for re-use and which is suitable for sterilization by radiation, electron beams, or gases.

In accordance with the objectives of the invention, a mechanically actuated non-reversible fluid injector device is provided in accordance with claim 1. The device includes an injection vial which is a double-ended cartridge, having a first end and a second end, the cartridge having a cylindrical bore defined by a wall extending between the first end and the second end, the bore being suitable for storing a fluid charge to be expelled therefrom. A first piston and a second piston are positioned within the bore and are slidably seated against the cylindrical wall of the bore, the first piston comprising means for reversibly engaging a plunger. The cartridge is provided with a puncturable end cap which is rigidly attached and sealed to the second end of the cartridge and which comprises a needle guide. The cartridge is also provided with a hollow injection needle having an external tip and an internal tip, the internal tip being rigidly attached to and passing through the second piston, and the external tip extending toward the end cap without protruding therefrom, and means for locking the first piston and the second piston irreversibly together when the first piston and the second piston are a predetermined distance apart. It is preferred that the puncturable end cap be made of a self-sealing material, although this is not necessary for practicing the invention.

The vial may also be provided with a readable indicator to indicate the type of medication, patient information, the amount of medicine to be injected and the various rates of needle insertion and withdrawal and of fluid injection and/or aspiration.

In accordance with another aspect of the invention, a programmable automatic injection/aspiration device is provided having a housing, a chamber disposed within the housing for reversibly receiving a cartridge vial, and a cartridge vial piston operating plunger slidably disposed within the housing. Also provided are means, disposed within the housing, for propelling the plunger. Similarly, controller means for controlling the rate, direction, and extent of movement of the plunger are provided. Control is thus provided via said propelling means, and a programmable processor for directing the controller, a sensor for detecting the rate, direction, and extent of movement of the plunger, an indicator for indicating the rate, direction, and extent of movement of the plunger and for indicating the amount of fluid remaining and expelled from a fluid vial, and a sensor for detecting the presence in the vial of aspirated physiological fluids are also provided. Similarly provided are means for automatically preventing injection into an undesirable target tissue upon the detection of aspirated physiological fluid, and locking means for preventing unintentional discharge of drug from a cartridge vial disposed in the injection/aspiration device.

In accordance with the invention a cartridge vial, containing a fluid medicine charge, may be inserted into an injection/aspiration device, which may then be used to administer the medicine by injection. Finally, the cartridge vial, with completely retracted needle, may be removed and discarded, while the injection/aspiration device may be repeatedly re-used with different cartridge vials.

In accordance with other objects of the invention, a mechanically actuated cartridge vial for fluid injection is provided. The mechanically actuated cartridge vial is a cartridge, having a first end and a second end, the cartridge having a cylindrical bore which is widened at the second end of the cartridge to form a cylindrical needle housing assembly residence chamber for reversibly receiving a needle housing assembly, and a puncturable end cap, the end cap sealing the second end of the cartridge and having a needle guide disposed therein.

The cartridge has an ampule disposed within it, the ampule having a bore, and the bore having a first cylindrical portion, a second cylindrical portion adjacent to and narrower than the first portion, and a third cylindrical portion adjacent to the second portion and being adjacent to and wider than the second portion, the ampule being suitable for storing a fluid charge to be expelled therefrom. The ampule is provide with a piston within the first portion of the ampule and being slidably seated in the ampule and a puncturable ampule end stopper rigidly attached to and sealing the third portion of the ampule.

A needle housing assembly is disposed in the cylindrical bore of the cartridge, the needle housing assembly comprising a housing, a hollow injection needle rigidly attached to the housing and having an internal end and an external end, the internal end of the needle extending through the needle assembly housing toward the puncturable ampule end stopper, and the external end of the hollow injection needle extending toward the puncturable end cap of the cartridge without protruding therefrom; and an actuator for engaging the cartridge vial and for propelling the ampule toward the puncturable end cap and for propelling the piston within the ampule toward the ampule end stopper to inject a fluid.

The needle housing assembly disposed within the cartridge is provided with locking means for irreversibly locking the needle assembly housing to the ampule when the needle housing assembly and the ampule are a predetermined distance apart. The mechanical actuator component of the invention comprises an actuator housing, the housing having a first end and a second end, the first end adapted for receiving the cartridge vial and the second end adapted for operating the cartridge vial and actuator.

A piston driver is slidably seated in the housing, the driver having a piston face for contacting the ampule piston, a transfer surface for transferring propelling force from the housing to the driver, and a trigger end for releasably engaging the second end of the actuator housing. Also provided in the actuator housing are a detent in the second firing end for releasably engaging the piston driver, a detent release in the second firing end for disengaging the piston driver from the second end, and a propulsion mechanism disposed between the transfer surface and the second end of the housing for driving the piston driver toward the puncturable end cap when the detent release is triggered.

In accordance with yet further objects of the invention, the propulsion mechanism disposed in the mechanical actuator is a spring of one or more materials from the group consisting of metals, glasses, composites and plastics. Alternatively, the propulsion mechanism can be a compressed gas, such as air, nitrogen, carbon dioxide, or the like, releasably contained in a receptacle.

### Brief Description of the Drawings

The invention will now be described with reference to the accompanying drawings, in which:
Figure 1 is a longitudinal cross-sectional view of an injection/aspiration device showing the relative placement of the cartridge vial and other components;
Figure 2 is a longitudinal cross-sectional view of the cartridge vial constructed in accordance with a first embodiment of the invention which ampule can be manufactured using conventional pharmaceutical packaging machinery.
Figure 3 illustrates an additional embodiment of the cartridge vial similar to that shown in Figure 2 but for use with injections only.
Figure 4 is a longitudinal cross-sectional view of a cartridge vial device for use with a mechanically powered actuator showing the relative placement of the ampule, cartridge, needle housing assembly, and other components;
Figure 5 is a longitudinal cross-sectional view of a spring-powered cartridge vial actuator before insertion of a cartridge vial, and showing the relative positions of the actuator housing, piston driver, spring, and firing cap assembly of a preferred embodiment of the invention;
Figure 6 is a magnified view of the firing cap assembly shown in Figure 5 to more clearly show the details of the invention.
Figure 7 is a longitudinal cross-sectional view of a cartridge vial for use with a mechanically powered actuator mated to such an actuator and showing the relative placement of major components of the vial and actuator after firing of the actuator, completion of an injection ,and covering of the exposed injection needle by the actuator safety pin.

### Detailed Description of the Preferred Embodiments

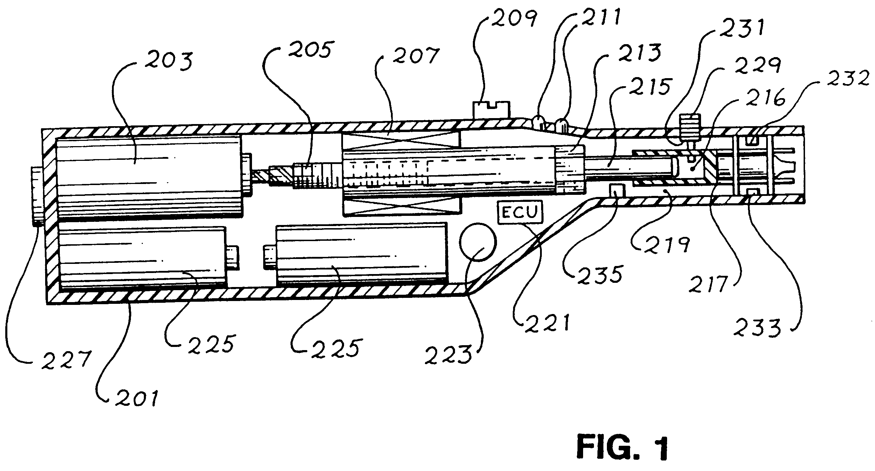

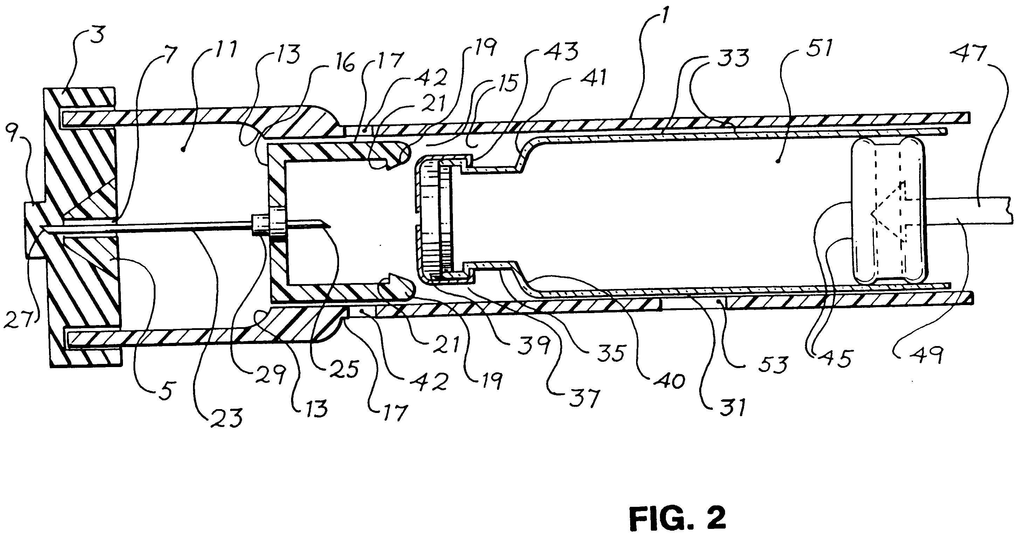

The injection/aspiration device, illustrated in Figure 1, comprises: a housing 201 for disposition of the various components having a chamber 219 for reversibly receiving a cartridge vial of a type as described below and in Figure 2 a d.c. motor 203, a lead screw 205, a linear bearing 207 for holding a lead nut, a lead nut **213** surrounding the lead screw, and armed/safety injector switch **209**, indicating light emitting diodes **211**, a plunger **215** attached to lead screw **205**, a cartridge vial **217** disposed within chamber **219**, an electronic control unit **221**, an injector trigger button **223**, a d.c. power source **225**, an on/off switch **227**, a cartridge/ampule position locking solenoid **229** having cartridge/ampule position locking solenoid shaft **231**, aspirated fluid sensor **233**, and plunger shaft rate/position/direction sensor **235**.

The injection/aspiration device of the invention operates in conjunction with cartridge vials as described hereinbelow. All of the components of the device are energized by d.c. power source **225** through the operation of switch **227**. D.c. motor **203** propels lead screw **205** which is attached to lead nut **213** and plunger shaft **215** which is reversibly attached to cartridge vial shaft end **216** by means of a mechanical key way provided at the end of plunger shaft **215**. Linear bearing **207** is provided for smooth operation of lead screw **205** and plunger shaft **215**. Lead screw nut **213** is provided as an attachment means for connecting lead screw **205** to plunger shaft **215**. Electronic control unit **221** is a programmable electronic processor connected by means of wires not shown to DC motor **203** to control the rate, direction, and extent of movement of lead screw **205**, plunger shaft **215** and, thereby, the rate, extent of movement, and direction of movement of cartridge vial shaft end **216**. Cartridge vial shaft end **216** can be of any type configured to securely but reversibly attach to plunger shaft **215**. Similarly, as described below, plunger shaft **215** can be adapted to operate other types of cartridge vials such as those designed to be used with a spear type shaft of a type known in the dental cartridge field. The electronic control unit **221** is programmed by means of a programmable read-only memory (PROM), and a programmable array logic (PAL) which, depending on the application of the device, can be interchanged to adapt the device to the age or sex of the patient, the location parameters of the injection, (such as intramuscular, subcutaneous or intravenous) for the rate of needle withdrawal and insertion, and for the rate of medicinal charge injection. The electronic control unit also comprises electronic timers which control the overall timing and specific rates during the injection process. A set of programmed instructions are stored in a replaceable and interchangeable memory chip (not shown) in electronic control unit **221**. By either interchanging memory chips or by reprogramming programmable memory chips, the various functions of the injection/aspiration device can be controllably varied. Cartridge/ampule position locking mechanism **229** is a solenoid which can be activated to lock a cartridge vial in a particular position by means of cartridge/ampule position locking solenoid shaft **231** so that blood or other fluids can be aspirated. Although a solenoid and solenoid shaft are described herein as a locking mechanism, it can be clearly seen that many other means for accomplishing the objective of the device to lock any type of cartridge vial in a particular position in the injection/aspiration device housing can be used.

Similarly, although programmable and/or interchangeable memory chips are described examples herein, it can be clearly seen that many other means for programming the stated functions could be used to accomplish the objectives of the invention.

It is also clear that although a d.c. motor and power source and electronic components are described herein as means for controlling the various rates of operation of the various components of the invention that other means such as compressed gas and valves could also be used.

Although not shown in Fig. 1, the various electronic and electrical components of the invention are connected electrical circuitry.

It should also be clear that many different means for securely connecting to and easily disengaging a cartridge vial to and from plunger shaft **215** are possible. As an example and not described as a limitation, Fig.2 shows plunger shaft **47** of an injection/aspiration device having pointed barbed plunger shaft head **49** similar to pointed shafts known in the dental injection cartridge field which is driven a sufficient distance into piston **45** during operation of the invention that a sufficiently secure connection is made to operate piston **45** in both directions. Disconnection of barbed head **49** from piston **45** is achieved by the retraction of plunger shaft **47** and plunger shaft head **49** a sufficient distance into lead nut **213** which is of an appropriate internal diameter to accomplish the disconnection.

The cartridge vial portion of the invention includes a hollow injection/aspiration needle attached to one piston with its external pointed end initially disposed within a sealed end cap for sanitary purposes. A second piston is also provided in the cylindrical bore to operate in conjunction with the first piston to impel the injection needle and the injection fluid into the subject and then withdraw the injection needle and first piston from the subject through the sealed end cap sufficiently so that the point of the needle need not be exposed to the atmosphere before, during, or after operation of the invention. The present invention provides a means for withdrawing the needle tip into a disposable cartridge immediately after the injection and before the injector device is withdrawn from contact with the subject. This reduces the risk of unintentional exposure to a used needle tip by both users and non-users because the needle tip need never be exposed.

The programmable automatic injector portion of the invention includes a chamber for insertion of a vial, a piston operating plunger for operation of the vial, and a power source for operating the plunger. The injector also includes a controller for varying the rate of needle insertion and withdrawal and the rate of fluid injection. The invention can be used to aspirate blood or other fluids.

### Cartridge Vial Embodiments:

The advantages and characteristics of the cartridge vial and injection/aspiration device according to the present invention can be elucidated from the following detailed description of one embodiment of the injection/aspiration device and three embodiments of the cartridge vial, to be taken as examples and not as limitations in conjunction with the accompanying drawings.

With reference to Fig. 2, a cartridge vial comprises a double-ended circularly cylindrical housing **1** constructed of rigid material and having a cartridge/ampule position locking solenoid shaft aperture **53**, aspirated fluid sensor apertures **42**, an ampule residence chamber **15**, and cylindrical needle housing assembly receiving chamber **11**. Cartridge housing **1** is provided with a puncturable sealing end cap **3** of resilient material, a plastic needle guide **5** rigidly embedded in the end cap **3** and having needle guide aperture **7** formed therein and an injection device positioning projection **9**. The needle assembly receiving chamber **11** is further provided with curved abutments **13** of appropriate configuration to allow the needle assembly housing **16** to both enter and withdraw from needle assembly housing receiving chamber **11**. Needle assembly housing **16** and ampule **31** are both initially disposed within ampule residence chamber **15**. A glass ampule **31**, positioned within the cartridge, is provided with a three portion chamber in which is stored a fluid charge **51**. The first portion **33** of ampule **31** freely communicates with second portion **35** and third portion **37**. Third portion **37** is provided with a puncturable ampule stopper **39** of a resilient material. An ampule piston chamber abutment **40** forms the transition between first ampule portion **33** and second ampule portion **35** and forms a stop for a piston **45** during operation of the invention. The external portion of the transition between first ampule portion **33** and second ampule portion **35** forms an ampule shoulder **41**. Ampule **31** is further provided with flange engaging lips **43** which are formed by the narrowing transition area between second chamber portion **35** and third chamber portion **37**.

Ampule **31** is further provided with a piston **45** of resilient material for reversibly engaging a pointed plunger shaft head **49** having a plunger shaft **47** of an injection/aspiration device. Such an ampule could be reversibly engaged to the injection/aspiration device by many other means, other than a piston like piston **45** as shown, such as a T-shaft and keyway or any other means providing secure reversible engagement of a cartridge device to an injection/aspiration device.

Needle assembly housing **16** is provided with a needle assembly housing bottom **18**, needle housing walls **17**, needle assembly housing flanges **19** and needle assembly housing lips **21** disposed for securely engaging ampule **31**. Needle assembly housing **16** is further provided with a hollow injection needle **23** which passes therethrough and has an internal tip **25** disposed toward puncturable ampule stopper **39** and an external tip **27** which passes through aperture **7** of needle guide **5** but does not protrude through sealing end cap **3**.

Needle assembly housing walls **17** are of sufficient length so that, in operation of the cartridge as described hereinbelow, third ampule portion **37** is captured by the engagement of needle assembly housing flange lips **21** by flange engaging step **43** of ampule **31** before needle internal end **25** contacts puncturable ampule end stopper **39** and when ampule **31** has been propelled a sufficient distance toward puncturable cartridge sealing end cap **3** that needle assembly housing **16** is fully within needle assembly housing receiving chamber **11**.

Although a bar code indicator is seen as an efficient means for labeling both the cartridge vial and injector/aspirator components of the invention, any other means or multiple means could be employed to label the invention for machine readable purposes and for human readable purposes. For instance, both components of the invention could be labeled concurrently with readable magnetic strips, braille bumps and alphanumeric symbols.

The above-described embodiment of the ampule type cartridge vial of the invention operates as described hereinbelow.

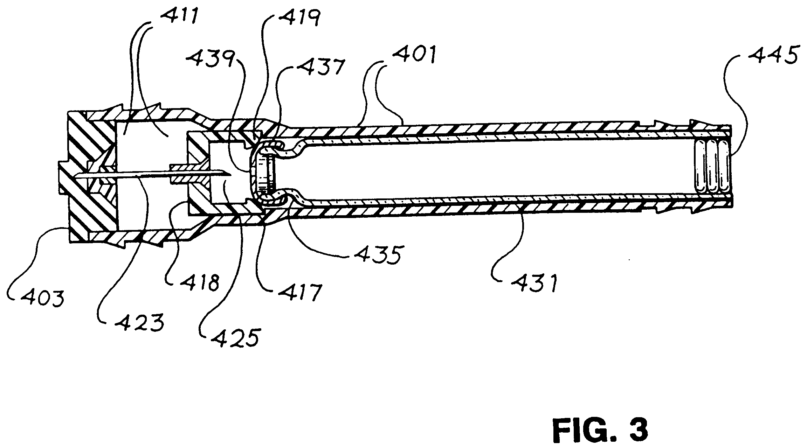

Piston **45** is propelled toward sealable end cap **3** by the application of force to plunger shaft **47** which force is communicated through plunger shaft head **49** to piston **45** thus applying hydraulic pressure through fluid charge **51** and forcing ampule **31** onto needle assembly **16**. Continued pressure on plunger shaft head **47** is communicated through needle housing flanges **19** needle housing walls **17** and needle housing bottom **16** to needle **23**, thus propelling needle **23** through needle guide aperture **7** causing external needle tip **27** to puncture puncturable end cap **3**. Continued force toward puncturable cartridge sealing end cap **3** by piston **45** continues to propel needle **23** outward until needle housing **16** is stopped by sealable end cap **3**, thus positioning needle assembly housing **16** within needle assembly receiving chamber **11** which is of a larger diameter than ampule residence chamber **15**. The positioning of needle assembly **16** within the needle assembly receiving chamber allows flanges **19** and walls **17** of needle assembly **16** to expand to irreversibly and securely receive and capture the third portion **37** of ampule **31**. Continued pressure in the same direction causes puncturable ampule stopper **39** on ampule **31** to be driven into internal needle tip **25** thus puncturing ampule stopper **39** and causing ampule stopper **39** to contact needle assembly housing bottom **18**. At this time in the operation of the invention, cartridge/ampule position locking solenoid **29** (shown in Fig. 1 but not shown in Fig. 3) is energized causing cartridge/ampule position locking solenoid shaft **31** (also shown in Fig. 2 but not shown in Fig. 3) to extend through cartridge/ampule position locking solenoid aperture **53** thus locking ampule **31** and needle assembly **17** tightly against needle guide **5**.

The direction of force on plunger shaft head **49** may then be reversed causing a slight withdrawal of piston **45** away from end cap **3**, thus causing aspiration into fluid **51** in ampule **31** of physio-logical fluid(s) if needle tip **27**, now located at a possible injection site in tissue, is in communication with such fluid. An aspirated fluid sensor **233** and light source, such as a photodiode or photocell **232** in the injection/aspiration component of the invention (shown in Fig. 1 but not in Fig. 3), then scan the fluid in ampule **31** through aspirated fluid sensor apertures **42** to spectrophotometrically detect the presence of physiological fluid(s).

If no fluids are detected and it is regarded as desirable or appropriate that none be aspiratable from an injection site for the drug being administered, or if fluid(s) that are detected do not indicate that the possible injection site, at which needle tip **27** is located is undesirable or inappropriate, the injection cycle continues and the site at which needle tip **27** is located, is employed as the injection site. Continued force in the same direction toward end cap **3** on piston **45** causes the expulsion of fluid charge **51** through needle **23** into the subject. In operation of the invention, with respect to an automatic injection/aspiration device of the type discussed above, the rate of propulsion of piston shaft head **49** is varied to control the rate of insertion of needle **23** and the rate of injection of fluid charge **51**. Also in the operation of the invention, a typical time for this sequence of inserting hollow injection needle **23** is on the order of 100 milliseconds. After the injection of fluid charge **51** the operational sequence is continued into the retraction/needle capture stage by the reversal of force on piston shaft head **49** thus causing pressure on piston **45** to be directed away from sealing cap **3**. The partial vacuum created by the withdrawal of piston **45** causes ampule **31** to retract from needle **23** a sufficient distance so that internal needle tip **25** no longer penetrates puncturable ampule end stopper **39**. The retraction of ampule **31** from needle tip **25** is assisted by the biasing force caused by the contact between curved needle assembly housing flanges **19** and curved ampule shoulders **41**. Thus, needle assembly housing flanges **21** are then engaged to flange engaging steps **43** on ampule **31**. Locking solenoid shaft **231** (shown in Figure 1 but not in Figure 3) is then withdrawn allowing further retention of the connected ampule/needle assembly. The ampule and needle assembly, thus irreversibly engaged, is withdrawn as piston **45** is withdrawn by continued force in the direction away from puncturable sealing end cap **3** thus withdrawing needle **23** from the injection site a sufficient distance so that external needle tip **27** is captured completely within needle assembly receiving chamber **11**. Thus, no part of needle **23** need ever be exposed to any environment other than the subject's tissues or the inside of the cartridge vial. A typical time for operation of the withdrawal/needle capture sequence is on the order of 200 milliseconds.

During operation of the invention, if an undesirable or inappropriate physiological fluid (e.g. blood) or lack of physiological fluid (e.g., lack of blood if an intravenous injection is intended) is detected by aspirated fluid sensor **233**, the injection sequence may be automatically stopped, thus preventing the injection of medicine or other fluids into an undesirable or inappropriate injection site.

With reference to Figure 3 , an embodiment of the cartridge vial of the present invention intended for injections where no aspiration is necessary is illustrated. Cartridge **401** is provided with slidable ampule **431** having piston **445** for the receipt of a barbed plunger shaft head known in the dental injection cartridge field and having a second ampule portion **435** which is smaller than third ampule portion **437** the transition area therebetween forming an engaging surface for engaging flanges **419** of needle housing assembly **418**. Needle assembly housing **418** is provided with needle assembly housing walls **417** of sufficient length that when the flanges capture third portion **437** of ampule **431** needle tip **425** has already punctured puncturable ampule end stopper **439**. It can thus clearly be seen that the retraction of needle assembly housing **418** and needle **423** are accomplished without permitting the withdrawal of needle tip **425** from puncturable end cap **439**.

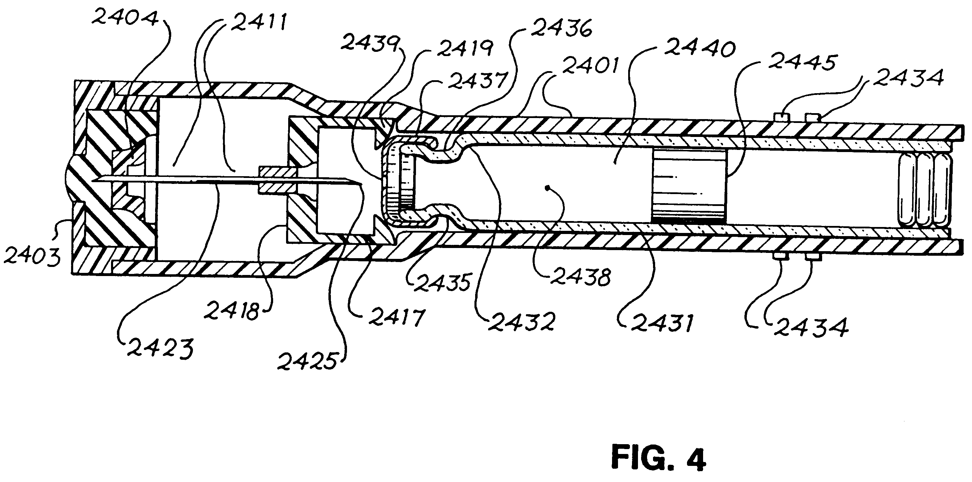

Although one preferred method of using the cartridge vials of the types described above is with a powered automatic injection/aspiration device also as described above, it can be clearly seen that a mechanically or manually powered injection device is preferable to operate such cartridge vials in some circumstances. For instance, where batteries or electrical power are unavailable or undesirable due to storage problems or where there is a need for a more compact, more simple, or less expensive device, a mechanical injector is preferred. Moreover, it is desirable to have an injector of which all the components can be stored for long periods of time, and which are suitable for sterilization by gamma or other radiation, electron beams, or gases such as ethlyene oxide. Figures 4-7 illustrate a preferred embodiment of a mechanical actuator and cartridge according to the invention.

With reference to Figure 4, an embodiment of the cartridge vial of the present invention intended for injections where no aspiration is necessary is illustrated. Cartridge **2401** is provided with slidable ampule **2431** having piston **2445** for expelling fluid from fluid chamber **2438** and having a second ampule portion **2435** which is smaller in outer diameter than third ampule portion **2437**, the transition area therebetween forming an engaging surface **2436** for engaging flanges **2419** of needle housing assembly **2418**. Needle assembly housing **2418** is provided with needle assembly housing walls **2417** of sufficient length that when the flanges **2419** capture third portion **2437** of ampule **2431** needle tip **2425** has already punctured puncturable ampule end stopper **2439**. Needle housing assembly walls **2417** are of a sufficient diameter such that expansion of walls **2417** cannot occur until needle housing assembly **2418** is pushed into needle housing assembly residence chamber **2411** by the movement of slidable ampule **2431**.

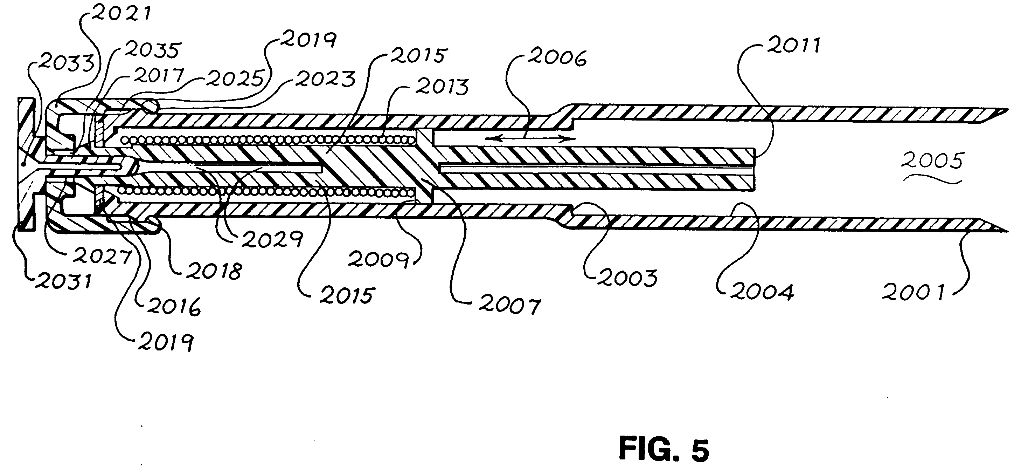

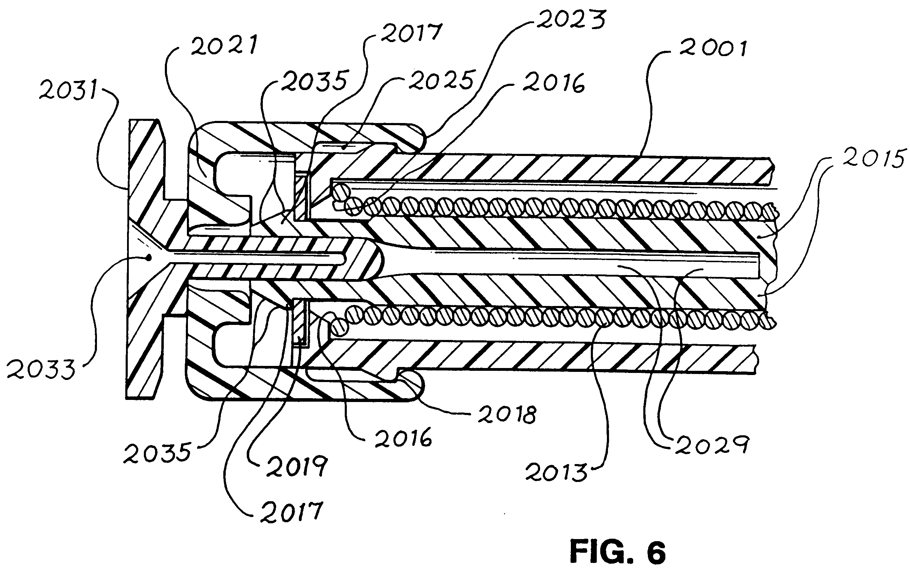

With reference to Figures 5 and 6, an embodiment of a cartridge vial actuator of the present invention intended for injections is illustrated. Cartridge vial actuator housing **2001** is provided with cartridge vial receiving chamber **2005** for receiving a cartridge vial of the type shown in Figure 4 and is also provided with cartridge vial locking indentations **2004** for engaging and positioning a cartridge vial. Cartridge vial actuator housing **2001** is also provided with cartridge vial positioning abutment **2003** for holding a cartridge vial in a predetermined position. Cartridge vial actuator housing **2001** is also provided with piston driver residence chamber **2006** wherein piston driver **2007** is slidably seated.

Piston driver **2007** is releasably attached through piston driver flange seat **2019** in the body of housing **2001** by piston driver release flanges **2017** which are attached to piston driver release arms **2015**. Piston driver **2007** is also provided with piston driver spring **2013** which is coiled around piston driver release arms **2015** in a compressed condition and abutting on piston driver spring shoulder **2009** and housing spring shelf **2016** thus biasing piston driver **2007** toward cartridge vial receiving chamber **2005**.

Cartridge vial actuator housing **2001** is also provided with firing cap **2021** having flange constriction surfaces **2035** which loosely abut piston driver release flanges **2017**. Firing cap **2021** is also provided with firing cap attachment lip **2023** which surrounds firing cap engagement ring **2018** and is further provided with firing cap slot **2025** which permits the longitudinal movement of firing cap **2021** with respect to cartridge vial actuator housing **2001**. Firing cap **2021** is also provided with firing cap safety pin aperture **2027** for receiving safety pin/needle cover **2031** therethrough.

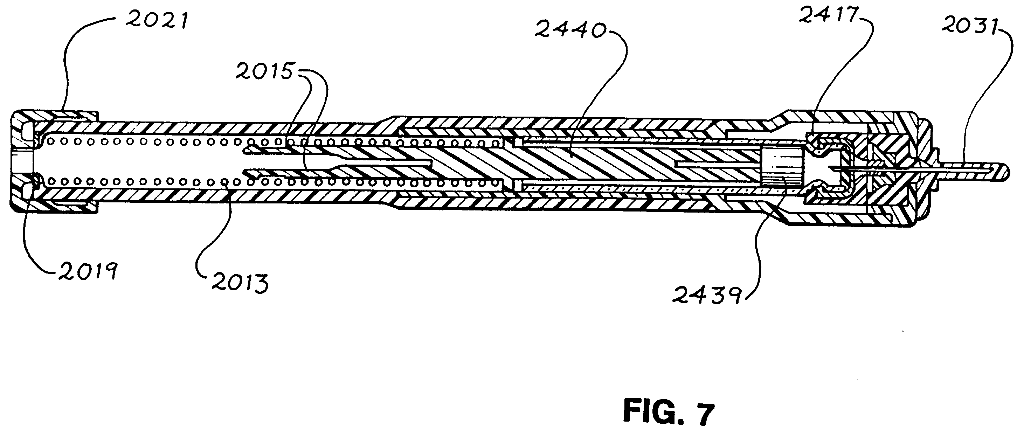

It can thus be clearly seen that piston driver release arms **2015** and piston driver flanges **2017** are held seated on piston driver flange seat **2019** by the expansion of piston driver safety pin void **2029** by safety pin/needle cover **2031**. Safety pin/needle cover **2031** is also provided with needle cover aperture **2033** which can be used to cover the protruding portion of injection needle **2423** after an injection, e.g. injection needle **2423** shown in Figure 4. See Figure 7.

The mechanically actuated cartridge vial of the invention, as shown in figures 4-7 , operates as described hereinbelow.

The space in ampule fluid chamber **2438** is filled with charge **2440** of fluid medicament to be injected into a subject. Cartridge vial housing **2401** is disposed within cartridge vial receiving chamber **2005** of cartridge vial actuator housing **2001** in a position determined by cartridge vial position abutment **2003** and cartridge vial locking indentations **2004** and cartridge vial locking projections **2434**.

The force of compressed piston driver spring **2013** is released by the following actions: safety pin/needle cover **2031** disposed within piston driver safety pin void **2029** is withdrawn from void **2029** through firing cap safety pin aperture **2027**. Firing cap **2021** is then depressed in the direction of cartridge vial receiving chamber **2005** thus causing angled flange constriction surfaces **2035** to impinge upon the angled surfaces of piston driver release flanges **2017** thereby causing the movement of those flanges toward each other and permitting flanges **2017** to be pulled through an aperture in piston driver flange seat **2019** by the force of expanding piston driver spring **2013** thus causing movement of piston driver **2007** in the direction of cartridge vial end cap **2403**. The movement of piston driver **2007** causes cartridge vial piston contact face **2011** to contact piston **2445** disposed above fluid chamber **2438** in ampule **2431** thus applying hydraulic pressure through fluid charge **2440** to propel slidable ampule **2431** toward end cap **2403**.

Thus, continued movement of piston driver **2007** causes movement of needle assembly housing **2418** also in the direction of end cap **2403** so that injection needle **2423** punctures end cap **2403** and needle assembly housing **2418** enters needle assembly housing residence chamber **2411** until the needle assembly housing is stopped by needle guide **2404** disposed within end cap **2403**. Continued pressure on piston **2445** then causes the expansion of needle assembly housing flanges **2419** and the puncture of puncturable ampule end stopper **2439** by needle internal end **2425**.

Continued pressure on piston **2445** by expanding piston driver **2013** communicated through piston driver **2007** causes ejection of fluid charge **2440** through hollow injection needle **2423** until piston **2445** bottoms out against ampule internal abutment **2432**. The mechanically actuated cartridge vial is then removed from a subject target area and safety pin/needle cover **2031** is then placed over the exposed portion of extended injection needle **2423** to prevent unwanted or unintentional punctures by the needle. Thus, no part of needle **2423** need ever be exposed to any environment other than the subject's tissues or the inside of the cartridge vial before injection.

A preferred material for the actuator housing, cartridge housing, needle assembly housing, firing cap, needle guide, end cap, safety pin/needle cover, and piston driver is radiation resistant polypropylene known in the medical arts. However, it can clearly be seen that many other materials which are standard in the medical and dental packaging and hypodermic syringe art such as plastics, including glass-filled plastics, carbon fiber composites, rubber, synthetic and nonsynthetic materials known in the art and nylon/carbon fiber composites. A preferred material for the injection needle and piston driver flange seat is stainless steel. However, any other reasonable substitute material such as glass, ceramics, and carbon fiber composites are acceptable.

Similarly, a preferred material for the piston driver is a nylon/carbon fiber composite, although numerous other materials having sufficient resilience, rigidity, and tensile strengths are acceptable to practice the present invention.

Preferred materials for the ampule piston, cartridge end cap, and puncturable shield include natural or synthetic rubber, silicone rubber, glass-filled silicone rubber, and various plastics so long as the desired characteristics of sealability, puncturability, and manufacture to precise tolerances are achievable. A preferred material for the puncturable ampule end stopper is a rubber disk or membrane securely attached and sealed to the ampule by an aluminum cover as is standard in the medical and dental arts.

A preferred material for the ampule is silicone treated USP glass, although many plastics and composite materials are also suitable as known in the art.

Suitable materials for the piston driver spring also include carbon steel, stainless steel, glass-filled nylon, various glasses, or any other material capable of providing biasing and propelling force in the context of the invention.

It is preferred that all components of the invention be amenable to sterilization by processes using gamma rays, electron beams, or ethylene oxide. Such materials include ABS plastics, polycarbonates, glasses and carbon fiber composites.

The needle assembly housing dimensions must be tailored with respect to the specific material used in order to achieve acceptable deformation characteristics to permit expansion of the needle assembly housing flanges under the particular amount of force transmitted from the piston driver spring. Of course, it can also be clearly seen that spring characteristics such as uncompressed length, material, and stiffness can be varied to adapt the piston driver spring for use with particular types of ampule and needle housing assemblies.

With slight modifications, the present invention can be adapted to be driven by forces other than the release of a compressed spring. For example, a device of the invention can be driven by release of a compressed gas releasably contained in a receptacle substituted for the piston driver arms **2015** and spring **2013** as shown in Figures 6 and 7. Release of the gas would then drive piston **2445** toward end cap **2403** of cartridge vial housing **2401** seated in receiving chamber **2005** to thus operate the invention in a manner similar to that of the spring-actuated cartridge described above.

Of course, it is also clear that, with slight modification of the present invention, the device can be configured to permit retraction of the needle, after injection, to reside wholly within the cartridge vial.

In a further adaptation, some portion of the trigger end of piston driver **2007** could be made frangible to the action of the detent release. For example, driver flanges **2017** could be made of a brittle plastic or composite which, upon depresssion of firing cap **2031**, break away from release arms **2015** to release the energy of spring **2013**. Thus, the device can be made to be non-reusable to prevent unsanitary or unwanted use. Examples of materials of which the frangible component can be made include glass-filled composites, metal-filled composites, carbon fiber composites, fiberglass, glasses, nylon, nylon/carbon fiber composites, and wood.

For example, by providing barbs or other means for irreversibly engaging the ampule piston to the piston driver, and by providing means whereby the actuator assembly can be disengaged from the cartridge vial in a manner which allows withdrawal of the piston driver from the ampule, retraction of the injection needle to reside wholly within the cartridge can be accomplished.

It can also be clearly seen that both the injector and the injector/aspirator of the present invention are suitable for use with cartridges of the type disclosed in WO93/02720. Suitable modifications in the chamber for receipt of a cartridge may be necessary in order to adapt the present devices for use with some of the two-component cartridges disclosed in WO93/02720. However, these modifications are seen to be within the scope of the present invention and can be readily carried out by the skilled in the art. These cartridges are adapted for the storage of two components, at least one of which is a fluid, to form a fluid, injectable medicament immediately prior to injection and require a force applied to a piston for actuation.

With reference to the resilient materials disclosed herein, such materials are those standard in the medical and dental packaging and hypodermic syringe art such as rubber, plastics, and other synthetic and non-synthetic materials known in the art for accomplishing similar and related objectives. Also, all of the embodiments of the cartridge vials disclosed herein are adapted to be manufactured by standard medical and dental ampule and container manufacturing equipment.

Similarly, the cartridge vials disclosed herein are adapted to contain, when fully charged, from 0.1 to 100 milliliters of fluid. However, more typically such vials will contain, when fully charged, from 0.5 to 10 milliliters of fluid.

The needles will have lengths and gauges, and will be made of materials, that are standard in the hypodermic syringe/needle art.

## Claims

1. A mechanically actuated non-reversible fluid injector device comprising:
(A) a tubular cartridge (1), having a first end and a second end, said cartridge having an interior bore (15) which is widened at said second end of said cartridge to form a cylindrical needle housing assembly residence chamber (11) for reversibly receiving a needle housing assembly, and having a puncturable end cap (3) sealing said second end of said cartridge(1);
(B) an ampule (31) slidably disposed within and proximal to the narrow end of said cartridge bore (15), said ampule (31) having a first elongated cylindrical portion (33), a second cylindrical portion (35) adjacent to and narrower than said first portion (33), and a third cylindrical portion (37) adjacent to and wider than said second portion (35), said first portion (33) being distal to said residence chamber (11) and said third portion (37) being proximal to said residence chamber (11);
(C) a piston (45) slidably seated within said first portion (33) of said ampule (31), said piston (45) engageable with a fluid charge (51) stored in said ampule (31)and propellable toward said residence chamber (11) by a piston driver (47);
(D) a puncturable ampule end stopper (39) rigidly attached to and sealing the end of said third portion (37) of said ampule (31), said ampule (31), piston (45) and puncturable end stopper (39) enclosing a space which is suitable for storing a fluid charge (51) intended for injection;
(E) a needle housing assembly capable of slidably moving within said interior bore, said assembly disposed within said narrow end of said tubular cartridge, proximal to said residence chamber (11) prior to actuation and propellable into said residence chamber (11) upon actuation, said needle housing assembly comprising:
a housing (16), said housing (16) having locking means (21) for irreversibly locking said needle housing assembly to the third portion (37) of said ampule (31) when said needle housing assembly and said ampule (31) are brought together within said needle housing assembly residence chamber(11), and
a hollow injection needle (23) fixed to said housing (16) and having an internal end (25) and an external end (27), said internal end (25) extending through said needle housing assembly toward said puncturable ampule end stopper (39), and said external end (27) extending toward said puncturable end cap (3) of said cartridge (1) without protruding therefrom; and
(F) a fireable mechanical actuator rigidly engageable with said tubular cartridge, having an actuatable propulsion mechanism, and having the piston driver (47) engageable with said propulsion mechanism and with said piston (45) for exerting force against said piston (45),
whereby upon actuation, said piston driver propels said piston (45) and said ampule (31), when charged with fluid (51) intended for injection, toward said residence chamber (11), thereby propelling said ampule (31) into contact with said needle housing assembly, causing said needle housing assembly to slide from said narrow end of said bore (15) into said residence chamber (11) and causing said external end (27) of said needle (23) to puncture said end cap (3) and protrude therefrom, further propelling said ampule (31) toward said needle housing assembly causing said locking means (21) irreversibly to lock said needle housing assembly to said ampule (31) and said internal end (25) of said needle (23) to puncture said ampule end stopper (39), and finally propelling said piston (45) causing said fluid charge (51) to be injected through said external end (27) of said needle (23).

2. A fluid injector in accordance with claim 1, wherein said needle housing assembly further comprises a generally cup-shaped housing (16) having a housing bottom (18) and flexible housing wall sections (17) with two ends, each section fixed to the periphery of said housing bottom (18) at one end, and having a flange (19) with a lip (21), comprising said locking means, at said second end, said housing wall sections (17) extending towards said ampule (31),
wherein said hollow injection needle (23) is fixed to said housing bottom (18), the internal end (25) of said needle (23) extending from said housing bottom (18) between said housing wall sections (17) toward said puncturable ampule end stopper (39),
and wherein said flanges (19) of said housing wall sections (17) are engageable with said third portion (37) of said ampule (31) as said ampule (31) is propelled towards said needle housing assembly;
whereby upon actuation said piston driver (47) further propels said ampule (31) within said cartridge bore (15) toward said residence chamber (11) thus engaging said flanges (19) of said housing wall sections (17) and propelling said needle housing assembly from said first end of said cartridge (1) into said residence chamber (11) allowing said flexible housing wall sections (17) to expand outwardly in said needle housing assembly residence chamber (11), the further movement of said ampule (31) causing said housing wall section lips (21) irreversibly to lock onto the third portion (37) of said ampule (31), and the internal end (25) of said hollow injection needle (23) to puncture said ampule end stopper (39) thereby allowing fluid (51) to be injected through the external end (27) of said hollow injection needle (23).

3. A device as claimed in claim 1, wherein said actuator comprises:
(i) an actuator housing (2001), said housing having a first end and a second end, said first end adapted for receiving said tubular cartridge and said second end adapted for operating said actuator;
(ii) a piston driver (2007) slidably seated in said housing (2001), said driver (2007) having a piston face for contracting said ampule piston, a transfer surface for transferring propelling force from said housing (2001) to said driver (2007), and a trigger end for releasably engaging said second end of said actuator housing (2001);
(iii) a detent in said second firing end for a releasably engaging said piston driver (2007);
(iv) a detent release in said second firing end for disengaging said piston driver (2007) from said second end;
(v) a propulsion mechanism (2013) disposed between said transfer surface and said second end of said housing for driving said piston driver (2007) toward said puncturable end cap when said detent release is triggered.

4. A device as claimed in claim 3, wherein said propulsion mechanism (2013) is a spring.

5. A device as claimed in claim 4, wherein said spring comprises one or more materials from the group consisting of metals, glasses, composites and plastics.

6. A device as claimed in claim 3, wherein said propulsion mechanism is a compressed gas releasably contained in a receptacle.

7. A device as claimed in claim 1, further comprising a readable indicator (211).

8. A device as claimed in claim 1, further comprising locking means (209) for preventing unintentional discharge of said device.

9. A device as claimed in claim 8, wherein said locking means (209) comprises a removable pin having a projection for preventing activation of said detent release.

10. A device as claimed in claim 9, wherein said locking means further comprises a recess of suitable dimension for covering and gripping said needle portion protruding from said puncturable end cap after an injection.

11. A device as claimed in claim 1, further comprising a readable indicator for matching cartridge vials and ampules to said actuator and for indicating patient information.

12. A device as claimed in claim 3, wherein said trigger end of said piston driver comprises a shaft frangible to the action of said detent release.

13. A device as claimed in claim 12, wherein said frangible shaft comprises one or more materials from the group comprising glass-filled composites, metal-filled composites, carbon fibre composites, fibreglass, glass, nylon, nylon-carbon fibre composite, and wood.

14. A device as claimed in claim 1, wherein said external end of said injection needle is disposed within said puncturable end cap of said cartridge a sufficient distance to provide a microbe-impermeable seal without protruding therefrom.

15. A device as claimed in claim 1, wherein said internal end of said injection needle is disposed within a puncturable shield a sufficient distance to provide a microbe-impermeable seal without protruding therefrom.

16. A device as claimed in claim 15, wherein said puncturable end cap of said cartridge is provided with one or more compounds from the group comprising antiseptics, antibiotics, antivirals and antimicrobials.

17. A device as claimed in claim 15, wherein said puncturable shield is provided with one or more compounds from the group comprising antiseptics, antibiotics, antivirals and antimicrobials.

## Patentansprüche

1. Mechanisch betätigtet irreversible Fluidinjektorvorrichtung, umfassend:
(A) eine rohrförmige Patrone (1), die ein erstes Ende und ein zweites Ende aufweist, wobei die Patrone eine Innenbohrung (15) aufweist, die an dem zweiten Ende der Patrone aufgeweitet ist, um eine zylindrische Nadelgehäusebaugruppenaufnahmekammer (11) zur reversiblen Aufnahme einer Nadelgehäusebaugruppe zu bilden, sowie eine durchstechbare Endkappe (3) aufweist, die das zweite Ende der Patrone (1) dichtet;
(B) eine Ampulle (31), die verschiebbar in und proximal zu dem engen Ende der Patronenbohrung (15) angeordnet ist, wobei die Ampulle (31) einen ersten langgestreckten zylindrischen Abschnitt (33), einen zweiten zylindrischen Abschnitt (35) benachbart zu und enger als der erste Abschnitt (33) sowie einen dritten zylindrischen Abschnitt (37) benachbart zu und weiter als der zweite Abschnitt (35) aufweist, wobei der erste Abschnitt (33) zu der Aufnahmekammer (11) distal ist und der dritte Abschnitt (37) zu der Aufnahmekammer (11) proximal ist;
(C) einen Kolben (45), der in dem ersten Abschnitt (33) der Ampulle (31) verschiebbar aufgenommen ist, wobei der Kolben (45) mit einer Fluidcharge (51) in Eingriff gebracht werden kann, die in der Ampulle (31) gespeichert ist, und durch einen Kolbentreiber (47) zu der Aufnahmekammer (11) getrieben werden kann;
(D) einen durchstechbaren Ampullenendstöpsel (39), der an dem Ende des dritten Abschnitts (37) der Ampulle (31) starr angebracht ist und dieses abdichtet, wobei die Ampulle (31), der Kolben (45) und der durchstechbare Endstöpsel (39) einen Raum umschließen, der zum Speichern einer für eine Injektion vorgesehenen Fluidcharge (51) geeignet ist;
(E) eine Nadelgehäusebaugruppe, die sich verschiebbar in der Innenbohrung bewegen kann, wobei die Baugruppe vor einer Betätigung in dem engen Ende der rohrförmigen Patrone proximal zu der Aufnahmekammer (11) angeordnet ist und bei einer Betätigung in die Aufnahmekammer (11) treibbar ist, wobei die Nadelgehäusebaugruppe umfaßt:
ein Gehäuse (16), wobei das Gehäuse (16) Arretiermittel (21) zum irreversiblen Arretieren der Nadelgehäusebaugruppe an dem dritten Abschnitt (37) der Ampulle (31) aufweist, wenn die Nadelgehäusebaugruppe und die Ampulle (31) in der Nadelgehäusebaugruppenaufnahmekammer (11) zusammengebracht sind, und
eine hohle Injektionsnadel (23), die an dem Gehäuse (16) befestigt ist und ein Innenende (25) und ein Außenende (27) aufweist, wobei sich das Innenende (25) durch die Nadelgehäusebaugruppe zu dem durchstechbaren Ampullenendstöpsel (39) erstreckt und wobei sich das Außenende (27) zu der durchstechbaren Endkappe (3) der Patrone (1) erstreckt, ohne davon vorzustehen; und
(F) ein aktivierbares mechanisches Betätigungsorgan, das mit der rohrförmigen Patrone starr in Eingriff gebracht werden kann, einen betätigbaren Aktiviermechanismus aufweist und den mit dem Aktiviermechanismus und dem Kolben (45) in Eingriff bringbaren Kolbentreiber (47) aufweist zum Ausüben einer Kraft gegen den Kolben (45),
wodurch bei einem Betätigen der Kolbentreiber den Kolben (45) und die Ampulle (31), wenn sie mit für eine Injektion vorgesehenem Fluid (51) gefüllt ist, zu der Aufnahmekammer (11) treibt, wodurch die Ampulle (31) in den Kontakt mit der Nadelgehäusebaugruppe getrieben wird, was bewirkt, daß sich die Nadelgehäusebaugruppe von dem engen Ende der Bohrung (15) in die Aufnahmekammer (11) verschiebt und was bewirkt, daß das Außenende (27) der Nadel (23) die Endkappe (3) durchsticht und davon vorsteht, ferner die Ampulle (31) zu der Nadelgehäusebaugruppe getrieben wird, was bewirkt, daß die Arretiermittel (21) die Nadelgehäusebaugruppe irreversibel an der Ampulle (31) arretieren und daß das Innenende (45) der Nadel (23) den Ampullenendstöpsel (39) durchsticht und schließlich der Kolben (45) getrieben wird, was bewirkt, daß die zu injizierende Fluidcharge (51) durch das Außenende (27) der Nadel (23) injiziert wird.

2. Fluidinjektor nach Anspruch 1, bei welchem die Nadelgehäusebaugruppe ferner ein im allgemeinen becherförmiges Gehäuse (16) umfaßt, das einen Gehäuseboden (18) und flexible Gehäusewandungsabschnitte (17) mit zwei Enden aufweist, wobei jeder Abschnitt mit dem Umfang des Gehäusebodens (18) an einem Ende befestigt ist und einen Flansch (19) mit einer Lippe (21) aufweist, umfassend das Arretiermittel bei dem zweiten Ende, wobei sich die Gehäusewandungsabschnitte (17) zu der Ampulle (31) erstrecken,
bei welchem die hohle Injektionsnadel (23) an dem Gehäuseboden (18) befestigt ist, wobei sich das Innenende (25) der Nadel (23) von dem Gehäuseboden (18) zwischen den Gehäusewandungsabschnitten (17) zu dem durchstechbaren Ampullenendstöpsel (39) erstreckt,
und bei welchem die Flansche (19) der Gehäusewandungsabschnitte (17) mit dem dritten Abschnitt (37) der Ampulle (31) in Eingriff gebracht werden können, wenn die Ampulle (31) zu der Nadelgehäusebaugruppe getrieben wird;
woduch bei einer Betätigung der Kolbentreiber (47) ferner die Ampulle (31) in der Patronenbohrung (15) zu der Aufnahmekammer (11) treibt, und somit die Flansche (19) der Gehäusewandungsabschnitte (17) ergreift und die Nadelgehäusebaugruppe von dem ersten Ende der Patrone (1) in die Aufnahmekammer (11) treibt, was ermöglicht, daß sich die flexiblen Gehäusewandungsabschnitte (17) in der Nadelgehäusebaugruppenaufnahmekammer (11) nach außen aufweiten, wobei die weitere Bewegung der Ampulle (31) bewirkt, daß sich die Gehäusewandungsabschnittslippen (21) irreversibel an dem dritten Abschnitt (37) der Ampulle (31) arretieren und das Innenende (25) der hohen Injektionsnadel (23) den Ampullenendstöpsel (39) durchsticht, wodurch ermöglicht wird, daß Fluid (51) durch das Außenende (27) der hohen Injektionsnadel (23) injiziert wird.

3. Vorrichtung nach Anspruch 1, bei welcher das Betätigungsorgan umfaßt:
(i) ein Betätigungsorgangehäuse (2001), wobei das Gehäuse ein erstes Ende und ein zweites Ende aufweist, wobei das erste Ende zur Aufnahme der rohrförmigen Patrone ausgeführt ist und das zweite Ende zum Betätigen des Betätigungsorgans ausgeführt ist;
(ii) einen Kolbentreiber (2007), der verschiebbar in dem Gehäuse (2001) aufgenomme ist, wobei der Treiber (2007) eine Kolbenfläche zum Kontakt haben mit dem Ampullenkolben, eine Übertragungsfläche zum Übertragen von Antriebskraft von dem Gehäuse (2001) auf den Treiber (2007) sowie ein Auslöserende zum lösbaren Ergreifen des zweiten Endes des Betätigungsorgangehäuses (2001) aufweist;
(iii) eine Festhaltevorrichtung in dem zweiten Aktivierungsende für ein lösbares Ergreifen des Kolbentreibers (2007);
(iv) einen Festhaltevorrichtungsauslöser in dem zweiten Aktivierungsende zum Entkoppeln des Kolbentreibers (2007) von dem zweiten Ende;
(v) einen Aktiviermechanismus (2013), der zwischen der Übertragungsfläche und dem zweiten Ende des Gehäuses angeordnet ist zum Antrieb des Kolbentreibers (2007) zu der durchstechbaren Endkappe, wenn der Festhaltevorrichtungsauslöser ausgelöst wird.

4. Vorrichtung nach Anspruch 3, bei welcher der Aktiviermechanismus (2013) eine Feder ist.

5. Vorrichtung nach Anspruch 4, bei welcher die Feder ein oder mehrere Materialien aus der Gruppe umfaßt, die aus Metallen, Gläsern, Verbundstoffen und Kunststoffen besteht.

6. Vorrichtung nach Anspruch 3, bei welcher der Aktiviermechanismus ein komprimiertes Gas ist, das in einem Behältnis freisetzbar enthalten ist.

7. Vorrichtung nach Anspruch 1, ferner umfassend eine ablesbare Anzeigeeinrichtung (211).

8. Vorrichtung nach Anspruch 1, ferner umfassend Arretiermittel (209) zum Verhindern einer unerwünschten Entleerung der Vorrichtung.

9. Vorrichtung nach Anspruch 8, bei welcher die Arretiermittel (209) einen entfernbaren Stift umfassen, welcher einen Fortsatz aufweist zum Verhindern einer Aktivierung des Festhaltevorrichtungsauslösers.

10. Vorrichtung nach Anspruch 9, bei welcher die Arretiermittel ferner eine Ausnehmung von geeigneter Abmessung zum Abdecken und Greifen des Nadelabschnitts umfassen, der nach einer Injektion von der durchstechbaren Endkappe vorsteht.

11. Vorrichtung nach Anspruch 1, ferner umfassend eine ablesbaren Anzeigeeinrichtung zum Anpassen von Patronenfläschchen und Ampullen an das Betätigungsorgan und zum Anzeigen von Patienteninformationen.

12. Vorrichtung nach Anspruch 3, bei welcher das Auslöserende des Kolbentreibers einen Schaft umfaßt, der durch die Einwirkung des Festhaltevorrichtungsauslösers gebrochen werden kann.

13. Vorrichtung nach Anspruch 12, bei welcher der brechbare Schaft ein oder mehrere Materialien aus der Gruppe umfaßt, welche glashaltige Verbundstoffe, metallhaltige Verbundstoffe, Kohlenstoff-Faserverbundstoffe, Glasfaser, Glas, Nylon, Nylon-Kohlenstoff-Faserverbundstoff und Holz umfaßt.

14. Vorrichtung nach Anspruch 1, bei welcher das Außenende der Injektionsnadel in der durchstechbaren Endkappe der Patrone in einem ausreichenden Abstand angeordnet ist, um einen mikrobenundurchlässige Dichtung vorzusehen, ohne davon vorzustehen.

15. Vorrichtung nach Anspruch 1, bei welcher das Innenende der Injektionsnadel in einem durchstechbaren Schild in einem ausreichenden Abstand angeordnet ist, um eine mikrobenundurchlässigen Dichtung vorzusehen, ohne davon vorzustehen.

16. Vorrichtung nach Anspruch 15, bei welcher die durchstechbare Endkappe der Patrone mit einer oder mehreren Verbindungen aus der Gruppe versehen ist, die Antiseptika, Antibiotika, Antivirenmittel und Bakteriostatika umfaßt.

17. Vorrichtung nach Anspruch 15, bei welcher der durchstechbare Schild mit einer oder mehreren Verbindungen aus der Gruppe versehen ist, die Antiseptika, Antibiotika, Antivirenmittel und Bakteriostatika umfaßt.

## Revendications

1. Dispositif d'injection de fluide irréversible actionné mécaniquement comprenant :
(A) une cartouche tubulaire (1), comportant une première extrémité et une seconde extrémité, ladite cartouche comportant un alésage interne (15) qui est élargi à ladite seconde extrémité de ladite cartouche pour former une chambre de séjour d'ensemble de logement d'aiguille cylindrique (11) pour recevoir de manière réversible un ensemble de logement d'aiguille, et comportant un bouchon d'extrémité pouvant être perfore (3) fermant hermétiquement ladite seconde extrémité de ladite cartouche (1) ;
(B) une ampoule (31) disposée de manière à coulisser au sein et près de l'extrémité étroite dudit alésage de cartouche (15), ladite ampoule (31) comportant une première partie cylindrique allongée (33), une deuxième partie cylindrique (35) adjacente à, et plus étroite que, ladite première partie (33), et une troisième partie cylindrique (37) adjacente à, et plus large que, ladite deuxième partie (35), ladite première partie (33) étant distale relativement à ladite chambre de séjour (11) et ladite troisième partie (37), étant proximale relativement à ladite chambre de séjour (11) ;
(C) un piston (45) calé par coulissement au sein de ladite première partie (33) de ladite ampoule (31), ledit piston (45) pouvant être engagé avec une charge de fluide (51) stockée dans ladite ampoule (31) et pouvant être propulsé vers ladite chambre de séjour (11) par un organe de commande de piston (47) ;
(D) un obturateur d'extrémité d'ampoule pouvant être perforé (39) fixé rigidement à, et fermant hermétiquement, l'extrémité de ladite troisième partie (37) de ladite ampoule (31), ladite ampoule (31), le piston (45) et l'obturateur d'extrémité pouvant être perforé (39) délimitant un espace qui est adapté à stocker une charge de fluide (51) destinée à être injectée ;
(E) un ensemble de logement d'aiguille adapté à se déplacer par coulissement au sein dudit alésage interne, ledit ensemble disposé au sein de ladite extrémité étroite de ladite cartouche tubulaire, proximal de ladite chambre de séjour (11) avant l'actionnement et apte à être propulsé dans ladite chambre de séjour (11) lors de l'actionnement, ledit ensemble de logement d'aiguille comprenant :
un logement (16), ledit logement (16) comportant un moyen de verrouillage (21) pour verrouiller de manière irréversible ledit ensemble de logement d'aiguille à la troisième partie (37) de ladite ampoule (31) lorsque ledit ensemble de logement d'aiguille et ladite ampoule (31) sont mis en contact au sein de ladite chambre de séjour d'ensemble de logement d'aiguille (11), et
une aiguille d'injection creuse (23) fixée audit logement (16) et comportant une extrémité interne (25) et une extrémité externe (27), ladite extrémité interne (25) s'étendent dans ledit ensemble de logement d'aiguille vers ledit obturateur d'extrémité d'ampoule pouvant être perforé (39), et ladite extrémité externe (27) s'étendant vers ledit bouchon d'extrémité pouvant être perforé (3) de ladite cartouche (1) sans faire saillie depuis celui-ci ; et
(F) un organe d'actionnement mécanique à détente pouvant être engagé rigidement avec ladite cartouche tubulaire, comportant un mécanisme de propulsion à actionnement, et ayant l'organe de commande de piston (47) pouvant être engagé avec ledit mécanisme de propulsion et avec ledit piston (45) pour exercer une force à l'encontre dudit piston (45),
de sorte qu'au moment de l'actionnement, ledit organe de commande de piston propulse ledit piston (45) et ladite ampoule (31), lorsqu'elle est chargée de fluide (51) destiné à être injecté, vers ladite chambre de séjour (11), de manière à propulser ladite ampoule (31) en contact avec ledit ensemble de logement d'aiguille, amener ledit ensemble de logement d'aiguille à coulisser depuis ladite extrémité étroite dudit alésage (15) dans ladite chambre de séjour (11) et amener ladite extrémité externe (27) de ladite aiguille (23) à perforer ledit bouchon d'extrémité (3) et faire saillie depuis celui-ci, propulser davantage ladite ampoule (31) vers ledit ensemble de logement d'aiguille, ce qui amène ledit moyen de verrouillage (21) à verrouiller de manière irréversible ledit ensemble de logement d'aiguille à ladite ampoule (31) et ladite extrémité interne (25) de ladite aiguille (23) à perforer ledit obturateur d'extrémité d'ampoule (39), et enfin propulser ledit piston (45), ce qui amène ladite charge de fluide (51) à être injectée via ladite extrémité externe (27) de ladite aiguille (23).

2. Dispositif d'injection de fluide selon la revendication 1, dans lequel ledit ensemble de logement d'aiguille comprend, en outre, un logement généralement en forme de U (16) comportant un fond de logement (18) et des sections de parois de logement souples (17) avec deux extrémités, chaque section fixée à la périphérie dudit fond de logement (18) à une extrémité, et comportant un rebord (19) avec un bec (21), comprenant ledit moyen de verrouillage, à ladite seconde extrémité, lesdites sections de parois de logement (17) s'étendant vers ladite ampoule (31),
dans lequel ladite aiguille d'injection creuse (23) est fixée audit fond de logement (18), l'extrémité interne (25) de ladite aiguille (23) s'étendant depuis ledit fond de logement (18) entre lesdites sections de parois de logement (17) vers ledit obturateur d'extrémité d'ampoule pouvant être perforé (39),
et dans lequel lesdits rebords (19) desdites sections de parois de logement (17) sont adaptés à être engagés avec ladite troisième partie (37) de ladite ampoule (31) tandis que ladite ampoule (31) est propulsée vers ledit ensemble de logement d'aiguille ;
de sorte qu'au moment de l'actionnement ledit organe de commande de piston (47) propulse davantage ladite ampoule (31) au sein dudit alésage de cartouche (15) vers ladite chambre de séjour (11), ce qui engage lesdits rebords (19) desdites sections de parois de logement (17) et propulse ledit ensemble de logement d'aiguille depuis ladite première extrémité de ladite cartouche (1) dans ladite chambre de séjour (11), permettant aux dites sections de parois de logement souples (17) de se dilater vers l'extérieur dans ladite chambre de séjour d'ensemble de logement d'aiguille (11), la poursuite du déplacement de ladite ampoule (31) amenant lesdits becs (21) des sections de parois de logement à se verrouiller de manière irréversible sur la troisième partie (37) de ladite ampoule (31), et l'extrémité interne (25) de ladite aiguille d'injection creuse (23) à perforer ledit obturateur d'extrémité d'ampoule (39) de manière à permettre au fluide (51) d'être injecté via l'extrémité externe (27) de ladite aiguille d'injection creuse (23).

3. Dispositif selon la revendication 1, dans lequel ledit organe d'actionnement comprend :
(i) un logement d'organe d'actionnement (2001), ledit logement, comportant une première extrémité et une seconde extrémité, ladite première extrémité adaptée à recevoir ladite, cartouche tubulaire et ladite seconde extrémité adaptée à faire fonctionner ledit organe d'actionnement ;
(ii) un organe de commande de piston (2007) calé par coulissement dans ledit logement (2001), ledit organe de commande (2007) comportant une face de piston pour contacter ledit piston d'ampoule, une surface de transfert pour transférer la force de propulsion dudit logement (2001) audit organe de commande (2007), et une extrémité de déclenchement pour engager avec possibilité de libération ladite seconde extrémité dudit logement d'organe d'actionnement (2001) ;
(iii) un cliquet dans ladite seconde extrémité de détente pour engager avec possibilité de libération ledit organe de commande de piston (2007) ;
(iv) un organe de décliquetage dans ladite seconde extrémité de détente pour dégager ledit organe de commande de piston (2007) de ladite seconde extrémité ;
(v) un mécanisme de propulsion (2013) disposé entre ladite surface de transfert et ladite seconde extrémité dudit logement pour entraîner ledit organe de commande de piston (2007) vers ledit bouchon d'extrémité pouvant être perforé lorsque ledit organe de décliquetage est actionné.

4. Dispositif selon la revendication 3, dans lequel ledit mécanisme de propulsion (2013) est un ressort.

5. Dispositif selon la revendication 4, dans lequel ledit ressort comprend un ou plusieurs matériaux dans le groupe consistant en métaux, verres, composites et plastiques.

6. Dispositif selon la revendication 3, dans lequel ledit mécanisme de propulsion est un gaz comprimé contenu avec possibilité de libération dans un réceptacle.

7. Dispositif selon la revendication 1, comprenant, en outre, un indicateur visuel (211).

8. Dispositif selon la revendication 1, comprenant, en outre, un moyen de verrouillage (209) pour empêcher une décharge accidentelle dudit dispositif.

9. Dispositif selon la revendication 8, dans lequel ledit moyen de verrouillage (209) comprend une goupille amovible comportant une projection pour empêcher d'actionner ledit organe de décliquetage.

10. Dispositif selon la revendication 9, dans lequel ledit moyen de verrouillage comprend, en outre, un évidement de dimension appropriée pour couvrir et retenir ladite partie d'aiguille faisant saillie depuis ledit bouchon d'extrémité pouvant être perforé après une injection.

11. Dispositif selon la revendication 1, comprenant, en outre, un indicateur visuel pour assortir les flacons de cartouches et les ampoules audit organe d'actionnement et pour donner des renseignements sur le patient.

12. Dispositif selon la revendication 3, dans lequel ladite extrémité de déclenchement dudit organe de commande de piston comprend un axe qui se rompt lors de l'action dudit organe de décliquetage.

13. Dispositif selon la revendication 12, dans lequel ledit axe à rupture comprend un ou plusieurs matériaux dans la groupe consistant en composites chargés de verre, composites chargés de métal, composites de fibre de carbone, fibre de verre, verre, Nylon, composite Nylon-fibre de carbone, et bois.

14. Dispositif selon la revendication 1, dans lequel ladite extrémité externe de ladite aiguille d'injection est disposée au sein dudit bouchon d'extrémité pouvant être perforé de ladite cartouche à une distance suffisante pour obtenir un organe d'étanchéité imperméable aux microbes sans faire saillie depuis ledit bouchon.

15. Dispositif selon la revendication 1, dans lequel ladite extrémité interne de ladite aiguille d'injection est disposée ou sein d'un écran pouvant être perforé à une distance suffisante pour obtenir un organe d'étanchéité imperméable aux microbes sans faire saillie depuis ledit écran.

16. Dispositif selon la revendication 15, dans lequel ledit bouchon d'extrémité pouvant être perforé de ladite cartouche est doté d'un ou plusieurs composés faisant partie du groupe consistant en antiseptiques, antibiotiques, antiviraux et antimicrobiens.

17. Dispositif selon la revendication 15, dans lequel ledit écran pouvant être perforé est doté d'un ou plusieurs composés faisant partie du groupe consistant en antiseptiques, antibiotiques, antiviraux et antimicrobiens.
